Europäisches Patentamt

European Patent Office

Office européen des brevets

(19)

(11) Publication number: 0 379 338
A2

## EUROPEAN PATENT APPLICATION

(21) Application number: 90300450.5

(22) Date of filing: 16.01.90

(51) Int. Cl.5: C07K 7/10, C12N 15/16, A61K 37/36

(30) Priority: 17.01.89 US 297719

(43) Date of publication of application:
25.07.90 Bulletin 90/30

(84) Designated Contracting States:
AT BE CH DE DK ES FR GB IT LI LU NL SE

(71) Applicant: MERCK & CO. INC.
126, East Lincoln Avenue P.O. Box 2000
Rahway New Jersey 07065-0900(US)

(72) Inventor: Bayne, Marvin L.
131 Tudor Oval
Westfield, New Jersey 07090(US)
Inventor: Cascieri, Margaret A.
18 Charred Oak Lane
East Windsor, New Jersey 08520(US)

(74) Representative: Hesketh, Alan, Dr. et al
European Patent Department Merck & Co.,
Inc. Terlings Park Eastwick Road
Harlow Essex, CM20 2QR(GB)

(54) Human insulin-like growth factor analogs and their production in yeast.

(57) Synthetic genes encoding two 70-amino acid analogs of human insulin-like growth factor (hIGF-I) have been constructed and expressed in the yeast, Saccharomyces cerevisiae. The protein analogs, IGF 150 and IGF 125 contain the first 17 amino acids of the A chain of human insulin in place of residues 42 to 58 of hIGF-I, and residues 8-10 of the A chain in place of residues 49 to 51 of hIGF-I, respectively. The purified proteins have high affinity for the human type I IGF receptor (3.2 and 7.0 nM respectively) yet have reduced relative affinity for the human and murine 28 K IGF binding proteins (7 fold and 45 fold, respectively). IGF 150 is 100 times more active than normal hIGF-I in stimulating DNA synthesis in 3T3 cells, and should be a more active growth factor in vivo due to its reduced affinity for 28 K IGF binding proteins. The protein analogs thus have a variety of utilities such as in promoting lactation in animals; promoting growth and feed efficiency in animals; improving carcass quality by increasing lean and decreasing fat; promoting wound healing in animals, including humans; promoting glucose utilization in skeletal muscle, and stimulating erythropoiesis, the production of red blood cells.

FIGURE 1

EP 0 379 338 A2

# HUMAN INSULIN-LIKE GROWTH FACTOR ANALOGS WITH REDUCED BINDING TO 28 K IGF BINDING PROTEINS AND THEIR PRODUCTION IN YEAST

## BACKGROUND OF THE INVENTION

The incorporation of fragments of the insulin molecule into IGF-I has previously been attempted in the form of two-chain disulfide-linked insulin-like structures. These molecules have considerably reduced biological activity relative to IGF-I and serum carrier protein binding is still significant rendering the in vitro activity of such compounds of little in vivo utility. See Joshi et al. Biochemistry 24: 4208-42 (1985); DeVroede et al. Proc. Nat. Acad. Sci. U.S.A. 82: 3010-14 (1985); and Joshi et al. Biochem. and Biophys. Res. Comm. 133: 423-429 (1985). The IGF-I analogs described in this invention are produced as single chain IGF-I-like molecules with equal potency to IGF-I at the type I IGF receptor and reduced binding to 28 K IGF Binding Proteins rendering such analogs of significant potential in vivo utility.

## SUMMARY OF THE INVENTION

Human insulin-like growth factor I (hIGF-I, also called somatomedin C) is a 70-amino acid protein purified from human serum. It is believed to mediate many of the effects of growth hormone; in particular it has been demonstrated to stimulate growth in hypophysectomized rats. In addition, IGF-I has been shown to promote cell growth and differentiation of various cell types.

Human IGF-I shows a remarkable amino acid sequence homology to insulin. This homology is the basis of a computer generated three-dimensional structural model for hIGF-I. (Blundell et al. Proc. Natl. Acad. Sci. U.S.A. 75: 180-184 (1978) and Blundell et al. Fed. Proc. Am. Soc. Exp. Biol. 42: 2592-2597 (1983)). This model predicts that a portion of the insulin receptor binding region is conserved within the IGF-I molecule explaining the ability of hIGF-I to bind to insulin receptors. The model also suggests regions of hIGF-I molecule which may be responsible for binding to serum carrier proteins.

One of the major differences between hIGF-I and insulin is that in normal human blood, greater than 99% of the IGF-I is bound to serum carrier proteins which do not readily cross the capillary barrier. Thus most of the IGF in serum is inactive. In addition, IGF I, but not insulin, has high affinity for a 28 K IGF Binding Protein which is present in serum and amniotic fluid and which is secreted by most cells. The physiological significance of the IGF I-Binding Protein complex is not clear. However, binding proteins clearly play a role in modulating the activity of IGF. The presence of serum binding proteins is a barrier to the bioactivity and bioavailability of exogenously administered IGF-I.

Investigations into the role of these 28 K binding proteins in the bioactivity of IGF-I could lead potentially to important bioactive compounds. Our approach was to create a IGF-I analog that retains efficient binding to the type I receptor, yet would have reduced binding to these 28 K binding proteins. The design of these analogs is based on the observation that insulin does not bind to these proteins. A synthetic gene for human IGF-I was modified to encode two IGF-I analogs in which residues 42 to 58 of hIGF-I are replaced by the first 17 amino acids of the A chain of human insulin and in which residues 49-51 of hIGF I are replaced by residues 8-10 of the A chain of human insulin. The synthetic genes are then placed in a yeast recombinant DNA expression system and the peptide analogs which are produced by the modified yeast cells are extracted therefrom and purified.

Thus, it is an object of this invention to describe the preparation of synthetic genes encoding for IGF-I analogs and to describe the incorporation of such genes into a microorganism. A further object is to describe the preparation of the IGF-I analogs from culturing the genetically modified microorganism. A still further object of this invention is to describe the properties and uses of the IGF-I analogs thus prepared. Still further objects will become apparent from reading the following description.

## DESCRIPTION OF THE INVENTION

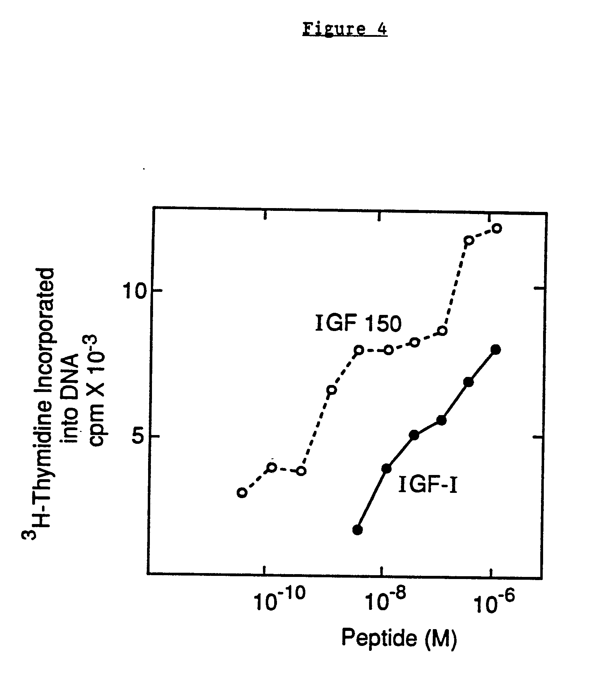

We have expressed synthetic genes encoding two 70-amino acid analogs of human IGF-I. These analogs, IGF150 and IGF125, contain the first 17 amino acids of human insulin A chain in place of residues 42 to 58 of hIGF-I, and residues 8-10 of the A chain of insulin in place of residues 49-51 of hIGF-I, respectively. These analogs have near equal affinity for the type I IGF receptor as compared to normal human IGF-I (Table 1). However, analogs IGF150 and IGF 125, have reduced binding to both human and rat 28K IGF binding proteins (Table 2). Thus, these new proteins retain nearly full activity at the type I IGF receptor but have reduced binding to 28 K IGF binding proteins. It is expected that these analogs will be more potent in vivo than normal IGF-I. IGF150 is 100 times more potent than normal IGF-I in stimulating DNA synthesis in 3T3 cells (Figure 4).

The synthetic genes of this invention encode for peptides which are analogs of human insulin-like growth factor (hIGF-I) and have the following structure where the letter designation for the constituent amino acids have the definitions given below:

IGF(1-40)-$A_1$-GIV-$A_2$-$A_3$-C-C-$A_4$-$A_5$-$A_6$-C-$A_7$-L-$A_8$-$A_9$-LE-R

wherein:

$A_1$ is T or I;

$A_2$ is D or E;

$A_3$ is E or Q;

$A_4$ is T;

$A_5$ is S;

$A_6$ is I;

$A_7$ is D or S;

$A_8$ is R or Y;

$A_9$ is R or Q; and

R is the remainder of the hIGF-I peptide consisting of 12 amino acids as follows:

MYCAPLKPAKSA

with the exception that the following gene:

IGF(1-40)-TGVIDECCFRSCDLRRLE-R which is the wild type hIGF-I and is excluded from the foregoing definition.

While the amino acid letter designations are generally well known to those skilled in the art, for purposes of clarity, the definitions as used herein are as follows:

A - Alanine

C - Cysteine

D - Aspartic acid

E - Glutamic acid

F - Phenylalanine

G - Glycine

H - Histidine

I - Isoleucine

K - Lysine

L - Leucine

M - Methionine

N - Asparagine

P - Proline

Q - Glutamine

R - Arginine

S - Serine

T - Threonine

V - Valine

Y - Tyrosine

Preferred variations of the foregoing peptide analogs are as follows:

$A_1$ is T or I;

$A_2$ is E;

$A_3$ is Q;

$A_4$ is T;

$A_5$ is S;

$A_6$ is I;

$A_7$ is S;

$A_8$ is Y; and

$A_9$ is Q.

Additionally, specific examples of such compounds are as follows:

IGF(1-40)IGIVEQCCTSICSLYQLE-R (Compound A or IGF150)

IGF(1-40)TGIVDECCTSICDLRRLE-R (Compound B or IGF125)

The peptide analogs can be produced by procedures similar to methods existing for the preparation of natural hIGF-I peptide, and modifications thereof which would be well-known to those skilled in the art. Specifically, these analogs may be synthesized chemically using procedures developed for human IGF-I. See for example Li et al. Proc. Natl. Acad. Sci. U.S.A. 80: 2216-2220 (1983). In accordance with the present invention the IGF-I analogs may also be produced following the transformation of susceptible bacterial, yeast or tissue culture cell hosts with recombinant plasmids that include DNA sequences capable of directing the expression of IGF-I analogs. The DNA sequence may be prepared synthetically, chromosomally, by recombinant DNA techniques or combination thereof. DNA sequences capable of directing the expression of IGF-I analogs could also be introduced into the germ line of animals or extra-chromasomally to produce transgenic animals endogenously producing the IGF-I analogs.

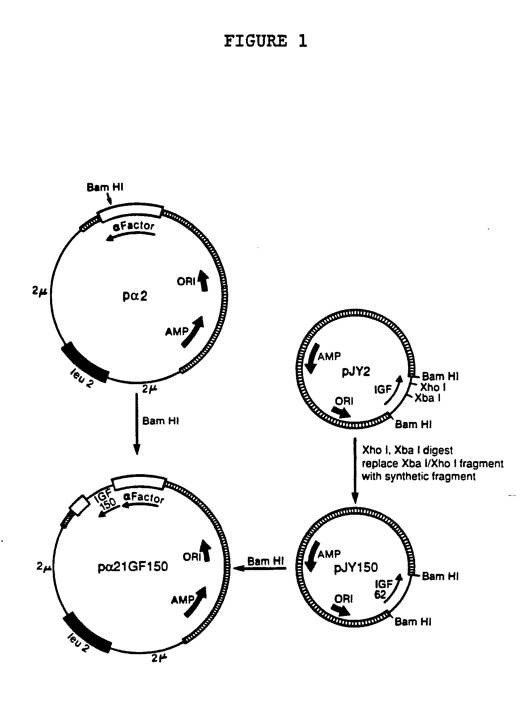

The synthetic genes of this invention are prepared using recombinant DNA biotechnology techniques well known to those skilled in the art. Figure 1 outlines the steps in combining the plasmids pα2 and PJY2 (Bayne et al Gene 66 235-244 (1988)) with the inclusion of the synthetic gene of this invention.

The instant synthetic gene produces analogs of hIGF-I which have substantial activity but, because they do not bind to 28 K IGF binding protein have levels of activity which, when taken on a molar or weight basis are considerably more active than wild-type hIGF-I. The compounds are thus highly active as agents to increase the yield and efficiency of milk production of animals, particularly ruminant animals such as cows. The compounds are also useful as growth promotant agents in food producing animals by increasing the rate of gain, feed efficency and carcass quality. The compounds are further useful as agents to promote wound healing and to stimulate erythropoiesis (the manufacture of red blood cells).

When used to increase milk production or as an animal growth promotant the compounds are administered parenterally such as by subcutaneous, intramuscular or intravenous injection or by a sustained release subcutaneous implant. In subcutaneous, intramuscular and intravenous injection the active ingredient is dissolved or dispersed in a liquid carrier vehicle. For parenteral administration, the active material is suitably admixed with an acceptable vehicle, preferably of the vegetable oil variety such as peanut oil, cotton seed oil and the like. Other parenteral vehicles such as organic preparation using solketal, glycerol, formal and aqueous parenteral formulations are also used. The active compound or compounds are dissolved or suspended in the parenteral formulation for administration; such formulations generally contain from 0.005 to 5% by weight of the active compound.

The instant compounds are effective by significantly increasing the level of milk production or the rate of weight gain or feed efficiency when administered at levels of from 0.1 to 100 mg per kg of animal body weight, preferably at from 1 to 10 mg/kg. When the compounds are administered in the form of a subcutaneous implant the compound is suspended or dissolved in a slowly dispersed material known to those skilled in the art, or administered in a device which slowly releases the active material through the use of a constant driving force such as an osmotic pump. In such cases constant administration over periods ranging from 20 to 120 days are possible with the active ingredient being released at from 0.1 to 10 mg/kg/day.

Because the hIGF-I analogs act synergistically with platelet-derived growth factor (PDGF) or other competence factors such as fibroblast growth factor (FGF) to stimulate DNA synthesis and cell replication in human fibroblasts, such analogs are useful to promote wound healing especially in cases where endogenous hIGF levels are low. Thus, the instant IGF-I analogs may be administered in combination with PDGF or FGF. The compounds could be administered parenterally, either subcutaneously, intramuscularly or intravenously using pharmaceutically acceptable parenteral formulation ingredients such as those listed above. The compounds would be administered at a dose of from 0.1 to 100 mg/kg, preferably from 1 to 10 mg/kg. Preferably, however, the compounds are administered topically when used as an agent to promote wound healing. Typical formulations for topical application are liquid, paste, ointment and spray formulations. The formulations could also be incorporated into a dressing which would be applied to the wound. The dressing would slowly release the compound directly to the site needing treatment.

The compounds would be incorporated into the topical formulation at concentrations of from 0.003 to 10% by weight with most formulations requiring from 0.3 to 3%. The concentration could be adjusted to provide for daily doses of from 0.06 to 2 mg of the active compound with allowance made to provide for multiple applications during any particular day.

The instant compounds may also be useful as erythropoietic agents possibly by virtue of their ability to stimulate late erythroid precursor differentiation. In such cases the compounds are administered parenterally as described above. The compounds may be administered either alone or in combination with erythropoietin to promote the production of red blood cells. For such uses the compounds are administered at doses of from 0.1 to 100 mg/kg, preferably from 1 to 10 mg/kg. Such doses are on a daily basis and if needed, the dose may be divided into multiple daily doses.

Attached hereto are figures which further describe and explain the instant invention.

Figure 1 describes the preparation of the recombinant plasmid pα2IGF150 from plasmid pα2 and plasmid pJY150 by selective cleavage and recombination. The plasmid encodes for the 70-amino acid analog of human IGF-I.

Figure 2A describes the DNA gene sequence and the analog it encodes which is inserted by ligation into plasmid pα2IGF150.

Figures 2B, similarly describes the DNA gene sequence and analogs for IGF125.

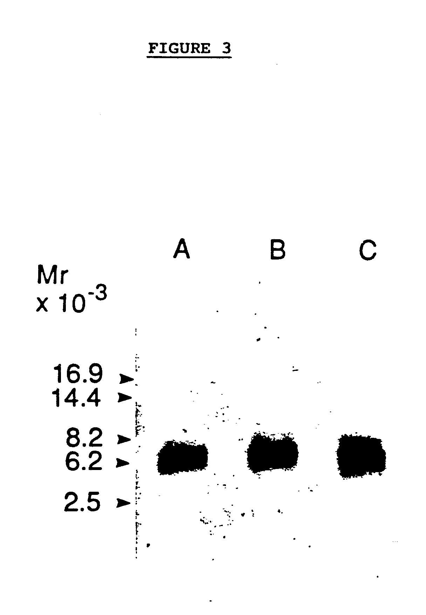

Figure 3 describes the silver stained gel after SDS- PAGE of IGF I (A) IGF125(B) and IGF150(C) after purification on Biogel P10 and high pressure liquid chromatography.

Figure 4 describes a comparison of biological activities of IGF-I with analog A (IGF150) in the ability to stimulate DNA synthesis in 3T3 cells. Analog A is observed to be 100 times more potent than wild-type IGF-I.

Table 1 describes the binding affinities of IGF I and analogs A (IGF150) and B(IGF125) for the human placental Type 1 IGF receptor.

Table 2 describes the binding affinities of IGF I and analogs A(IGF150) and B(IGF125) for the human and murine 3T3 cell 28 K IGF binding protein.


## EXAMPLE


## Construction of the IGF150 Analog Gene


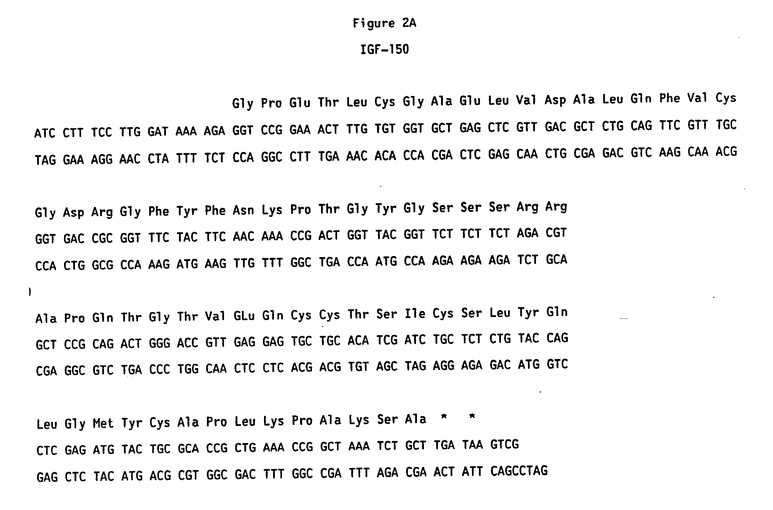

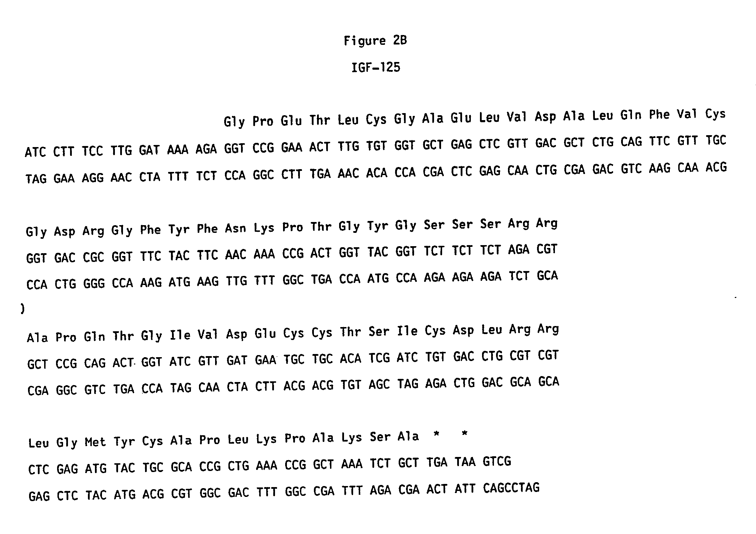

A synthetic gene encoding the 70 amino acids of hIGF-I has been assembled and cloned into pBR322 to yield plasmid phIGF. Plasmid phIGF was modified to form plasmid pJY2 (Bayne et al Gene 66 235-244-(1988)). Plasmid pJY2 was modified as described in Figure 1. Two oligonucleotides: IGF150-5′ CTAC ACGT GCT CCG CAG ACT GGG ATC GTT GAG CAG TGC TGC ACA TCG ATC TGC TCT CTG TAC CAGC 3′ and 5′- TCGA GCT GGT ACA GAG AGC AGA TCG ATG TGC AGC ACT GCT CAA CGA TCC CAG TCT GGC GGA GCA CGT-3′ were annealled to form a XbaI/XhoI replacement fragment. This fragment was inserted into pJY2 digested with endonuclease XbaI and XhoI. Transformation of E. coli with the ligation mixture yields bacteria carrying the plasmid pJY150. The DNA sequence and the analog IGF it encodes is shown in Figure 2A.


## Expression of Analog IGF150


The Bam HI IGF150 gene cassette from plasmid pJY150 was ligated into Bam HI digested pα2 as indicated in Figure 1. The plasmid with the IGF150 cassette in pα2 in the proper orientation was designated pα2IGF150. This plasmid was introduced into the yeast strain BJ1995. Yeast strain carrying the pα2IGF150 plasmid secrete the protein IGF150 into the growth media.


## Expression and Purification of Mutant hIGF I Peptides


Saccharomyces cerevisiae strain BJ1995 (MAT α, leu2, trp1, ura3, prb1-1122, pep4-3, ciro) was transformed with the appropriate expression plasmid and transformants were selected on leucine minus plates. Cells were grown to saturation in 1 liter of 5x leu(-) media, pH 4.8, containing 0.85% yeast nitrogen base without amino acids and ammonium sulfate supplemented with 4% glucose, 1% ammonium sulfate, 0.6% sodium hydroxide, 0.03% L-isoleucine, 0.03% L-phenylalanine, 0.025% L-tyrosine, 0.02% L-lysine, 0.02% L-tryptophan, 0.02% uracil, 0.02% adenine, 0.01% L-arginine, 0.005% methionine, 0.005% L-histidine, 29 µM ferric chloride, 25 µM zinc sulfate, and 1% succinic acid. Cells were removed by centrifugation at 3000 x g. The cleared supernatant was mixed with 10 g of BioRex 70 equilibrated in 1% succinic acid, pH 4.8. After stirring for 3 hours at 4° C, the resin was poured into a 2.5 cm column and washed with 1L of 1% succinic acid, pH 4.8. The peptide was eluted with 1M ammonium acetate, pH 8. Receptor active material was pooled, concentrated to 4 ml, then applied to a 2.5 x 90 cm Biogel P10 (200-400 mesh) column equilibrated in 1N acetic acid. Gel filtration was carried out at 30 ml per hour. Twelve ml fractions were collected and assayed for IGF-like activity by the radioreceptor assay. Active fractions were pooled and lyophilized. The activity was reconstituted in 0.2 ml 0.05% trifluoroacetic acid, 15% acetonitrile and loaded onto a C18 $\mu$Bondapak (0.46 x 25 cm, 10 micron, Waters) reverse phase HPLC column. The peptides were eluted from the column using a 15-50% acetonitrile gradient in 0.05% trifluoroacetic acid. The flow rate was 1 ml per minute and 1 minute fractions were collected and assayed by receptor assay. Active fractions were pooled and lyophilized. The purified peptide was quantitated by amino acid analysis and stored at -20°C in 0.1 N acetic acid at a concentration of 0.1 mM.

Characterization of IGF Analogs

Quantitative amino acid analysis was employed to determine the concentration of purified analogs. The amino acid composition is consistent with that expected for the analogs.

Affinity of the analogs to type I IGF receptor is shown in Table 1. Analog A (IGF150), and B (IGF125), inhibit the binding of $^{123}$I-hIGF-I to human placental membranes with a $IC_{50}$ of 3.2 nM, and 7.0 nM respectively, compared to 4.9 nM for wild type recombinant hIGF-I. Affinity of analogs A and B for human and murine 28 K IGF binding human proteins are shown in Table 2. Recombinant wild type hIGF-I inhibits binding of $^{123}$-hIGF-I to the 28 K human and murine IGF binding proteins with a $IC_{50}$ of 0.23 nM and 11.2 nM, respectively. Analog IGF150 inhibits this binding with a $IC_{50} > 1.5$ nM and 71 nM, respectively. Analog IGF125 inhibits this binding with $IC_{50} \gg 1.5$ nM and 501 nM, respectively.

IGF-I stimulates DNA synthesis in mouse 3T3 cells. As shown in Figure 4, IGF150 stimulates DNA synthesis in these cells with about 100-fold higher potency then wild type IGF-I.

## Claims

1. A synthetic polypeptide analog of hIGF-I which has the structure:
IGF(1-40)-$A_1$-GIV-$A_2$-$A_3$-CC-$A_4$-$A_5$-$A_6$-C-$A_7$-L-$A_8$-$A_9$-LE-R
wherein:
$A_1$ is T or I;.
$A_2$ is D or E;
$A_3$ is E or Q;
$A_4$ is T;
$A_5$ is S;
$A_6$ is I;
$A_7$ is D or S;
$A_8$ is R or Y;
$A_9$ is R or Q; and
R is the remainder of the hIGF-I peptide, provided that and $A_1$ to $A_9$ groups and the other amino acids do not constitute IGF(1-40)-TGVIDECCFRSCDLRRLE-R.

2. The peptide of Claim 1 wherein:
$A_1$ is T or I;
$A_2$ is E;
$A_3$ is Q;
$A_4$ is T;
$A_5$ is S;
$A_6$ is I;
$A_7$ is S;
$A_8$ is Y;
$A_9$ is Q.

3. The peptide of Claim 1 which is:
IGF(1-40)-IGIVEQCCTSICSLYQLE-R.

4. The peptide of Claim 1 which is:
IGF(1-40)-TGIVDECCTSICDLRRLE-R.

5. A synthetic gene encoding for the polypeptide of Claim 1.

6. A synthetic gene encoding for the polypeptide of Claim 3.

7. The synthetic gene of Claim 6 which is:

```
ATC CTT TCC TTG GAT AAA AGA GGT CCG GAA ACT TTG TGT
TAG GAA AGG AAC CTA TTT TCT CCA GGC CTT TGA AAC ACA

GGT GCT GAG CTC GTT GAC GCT CTG CAG TTC GTT TGC GGT
CCA CGA CTC GAG CAA CTG CGA GAC GTC AAG CAA ACG CCA

GAC CGC GGT TTC TAC TTC AAC AAA CCG ACT GGT TAC GGT TCT
CTG GCG CCA AAG ATG AAG TTG TTT GGC TGA CCA ATG CCA AGA

TCT TCT AGA CGT GCT CCG CAG ACT GGG ACC GTT GAG CAG TGC TGC
AGA AGA TCT GCA CGA GGC GTC TGA CCC TGG CAA CTC GTC ACG ACG

ACA TCG ATC TGC TCT CTG TAC CAG CTC GAG ATG TAC TGC GCA
TGT AGC TAG ACG AGA GAC ATG GTC GAG CTC TAC ATG ACG CGT

CCG CTG AAA CCG GCT AAA TCT GCT TGA TAA
GGC GAC TTT GGC CGA TTT AGA CGA ACT AGG
```

8. A synthetic gene encoding for the polypeptide of Claim 4.
9. The synthetic gene of Claim 8 which is:

```
ATC CTT TCC TTG GAT AAA AGA GGT CCG GAA ACT TTG TGT
TAG GAA AGG AAC CTA TTT TCT CCA GGC CTT TGA AAC ACA

GGT GCT GAG CTC GTT GAC GCT CTG CAG TTC GTT TGC GGT
CCA CGA CTC GAG CAA CTG CGA GAC GTC AAG CAA ACG CCA

GAC CGC GGT TTC TAC TTC AAC AAA CCG ACT GGT TAC GGT TCT
CTG GCG CCA AAG ATG AAG TTG TTT GGC TGA CCA ATG CCA AGA

TCT TCT AGA CGT GCT CCG CAG ACT GGT ATC GTT GAT GAA TGC
AGA AGA TCT GCA CGA GGC GTC TGA CCA TAG CAA CTA CTT ACG

TGC ACA TCG ATC TGT GAC CTG CGT CGT CTC GAG ATG TAC
ACG TGT AGC TAG ACA CTG GAC GCA GCA GAG CTC TAC ATG

TGC GCA CCG CTG AAA CCG GCT AAA TCT GCT TGA TAA
ACG CGT GGC GAC TTT GGC CGA TTT AGA CGA ACT ATT
```

10. A process for the preparation of the synthetic gene of Claim 1 which comprises:

a) the synthesis of the appropriate constituent oligonucleotides;

b) annealing and ligation of said oligonucleotides to gene fragments; and

c) cloning of synthetic gene into recombinant DNA plasmid.

11. A process for the preparation of the polypeptide analog of Claim 1 by the recombinant DNA expression systems of bacteria, yeast or tissue culture cell hosts which comprises:

a) insertion of the appropriate synthetic gene into an expression vector to form an expression cassette;

b) introduction of the expression cassette into the bacteria, yeast or tissue cell culture host;

c) growth of the transformed expression host; and

d) purification of the desired polypeptide analog from said host.

12. A method for the promotion of lactation in animals which comprises administering to a lactating animal a synthetic polypeptide analog of hIGF-I of Claim 1.

13. A composition useful for the promotion of lactation in animals which comprises an inert carrier and a synthetic polypeptide analog of hIGF-I of Claim 1.

14. The use of a synthetic polypeptide analog of hIGF-I of Claim 1, for the preparation of a composition useful for promoting growth and feed efficency in animals.

15. A composition useful for promoting growth and feed efficiency in animals which comprises an inert carrier and a synthetic polypeptide analog of hIGF-I of Claim 1.

16. A method for increasing the lean and decreasing the fat content of meat producing animals which comprises administering to such animals a synthetic polypeptide analog of hIGF-I of Claim 1.

17. A composition useful for increasing the lean and decreasing the fat content of meat producing animals which comprises an inert carrier and a synthetic polypeptide analog of hIGF-I of Claim 1.

18. The use of a synthetic polypeptide analog of hIGF-I of Claim 1, for the preparation of a composition useful for promoting wound healing in animals.

19. A composition useful for promoting wound healing in animals which comprises an inert carrier and a synthetic polypeptide analog of hIGF-I of Claim 1.

20. The use of a synthetic polypeptide analog of hIGF-I of Claim 1, for the preparation of a composition useful for stimulating erythropoiesis in animals.

21. A composition useful for stimulating erythropoiesis in animals which comprises an inert carrier and a synthetic polypeptide analog of hIGF-I of Claim 1.

FIGURE 1

Figure 2A

IGF-150


```
                    Gly Pro Glu Thr Leu Cys Gly Ala Glu Leu Val Asp Ala Leu Gln Phe Val Cys
ATC CTT TCC TTG GAT AAA AGA GGT CCG GAA ACT TTG TGT GGT GCT GAG CTC GTT GAC GCT CTG CAG TTC GTT TGC
TAG GAA AGG AAC CTA TTT TCT CCA GGC CTT TGA AAC ACA CCA CGA CTC GAG CAA CTG CGA GAC GTC AAG CAA ACG


Gly Asp Arg Gly Phe Tyr Phe Asn Lys Pro Thr Gly Tyr Gly Ser Ser Ser Arg Arg
GGT GAC CGC GGT TTC TAC TTC AAC AAA CCG ACT GGT TAC GGT TCT TCT TCT AGA CGT
CCA CTG GCG CCA AAG ATG AAG TTG TTT GGC TGA CCA ATG CCA AGA AGA AGA TCT GCA


Ala Pro Gln Thr Gly Thr Val GLu Gln Cys Cys Thr Ser Ile Cys Ser Leu Tyr Gln
GCT CCG CAG ACT GGG ACC GTT GAG GAG TGC TGC ACA TCG ATC TGC TCT CTG TAC CAG
CGA GGC GTC TGA CCC TGG CAA CTC CTC ACG ACG TGT AGC TAG AGG AGA GAC ATG GTC


Leu Gly Met Tyr Cys Ala Pro Leu Lys Pro Ala Lys Ser Ala  *   *
CTC GAG ATG TAC TGC GCA CCG CTG AAA CCG GCT AAA TCT GCT TGA TAA GTCG
GAG CTC TAC ATG ACG CGT GGC GAC TTT GGC CGA TTT AGA CGA ACT ATT CAGCCTAG
```

EP 0 379 338 A2

Figure 2B

IGF-125

```
                            Gly Pro Glu Thr Leu Cys Gly Ala Glu Leu Val Asp Ala Leu Gln Phe Val Cys
ATC CTT TCC TTG GAT AAA AGA GGT CCG GAA ACT TTG TGT GGT GCT GAG CTC GTT GAC GCT CTG CAG TTC GTT TGC
TAG GAA AGG AAC CTA TTT TCT CCA GGC CTT TGA AAC ACA CCA CGA CTC GAG CAA CTG CGA GAC GTC AAG CAA ACG


Gly Asp Arg Gly Phe Tyr Phe Asn Lys Pro Thr Gly Tyr Gly Ser Ser Ser Arg Arg
GGT GAC CGC GGT TTC TAC TTC AAC AAA CCG ACT GGT TAC GGT TCT TCT TCT AGA CGT
CCA CTG GGG CCA AAG ATG AAG TTG TTT GGC TGA CCA ATG CCA AGA AGA AGA TCT GCA
)
Ala Pro Gln Thr Gly Ile Val Asp Glu Cys Cys Thr Ser Ile Cys Asp Leu Arg Arg
GCT CCG CAG ACT· GGT ATC GTT GAT GAA TGC TGC ACA TCG ATC TGT GAC CTG CGT CGT
CGA GGC GTC TGA CCA TAG CAA CTA CTT ACG ACG TGT AGC TAG AGA CTG GAC GCA GCA


Leu Gly Met Tyr Cys Ala Pro Leu Lys Pro Ala Lys Ser Ala  *    *
CTC GAG ATG TAC TGC GCA CCG CTG AAA CCG GCT AAA TCT GCT TGA TAA GTCG
GAG CTC TAC ATG ACG CGT GGC GAC TTT GGC CGA TTT AGA CGA ACT ATT CAGCCTAG
```

FIGURE 3

Figure 4